# EUROPEAN PATENT APPLICATION

(11) **EP 1 674 468 A1**
(43) Date of publication of application: **28.06.2006**
(21) Application number: 04030234.1
(22) Date of filing: 21.12.2004
(51) Int. Cl.: C07D 495/04, A61K 31/4365, A61P 7/02

(54) **Polymorphs of clopidogrel hydrobromide**

(71) Applicant: Ratiopharm GmbH, 89079 Ulm (DE)
(72) Inventor: Jetti, Ram K.R., Dr., 6020 Innsbruck (AT); Griesser, Ulrich, Dr., 6020 Innsbruck (AT)
(74) Representative: Best, Michael

(57) **Abstract**

The invention relates to five anhydrous and two hydrated modifications of clopidogrel hydrobromide, their preparation and their use.

## Description

The present invention relates to the solid state chemistry of clopidogrel hydrobromide, a known antithrombotic drug. In particular, the present invention relates to certain polymorphs of clopidogrel hydrobromide, their preparation and use.

Arteriosclerosis is the buildup of plaque in the wall of the arteries leading to a thickening and a reduction in elasticity of the arteries. Arteriosclerosis results from injury to the inside layer of the artery. The injury is caused by common activities and diseases such as high cholesterol, high blood pressure, smoking and infection.

Plaques form on the inner walls of the artery at these sites of injury. The plaques are mainly composed of fatty tissue and smooth muscle cells. The formation of plaque often leads to blood clotting due to platelet aggregation at the site of the injury. This clotting may result in a reduction or elimination of blood flow to vital organs, causing heart attacks or other serious conditions. The plaque may also rupture and send a blood clot through the artery, referred to as an embolus, which if deposited in a smaller blood vessel may completely block blood flow.

Antiplatelet activity is desirable in fighting the often fatal results of arteriosclerosis, such as ischemic strokes, heart attacks or claudication.

Clopidogrel is an inhibitor of induced platelet aggregation which acts by inhibiting the binding of adenosine diphosphate to its receptor. Clopidogrel is metabolized by the liver into its active form. Its antiplatelet activity is extended in that it stops any platelet activity even up to ten days after administration.

The chemical name of clopidogrel is (αS)-α-(2-chlorophenyl)-6,7-dihydrothieno[3,2-c]-pyridine-5(4*H*)-acetic acid methyl ester (also known as methyl-(+)-(S)-(o-chlorophenyl)-6,7-dihydrothieno[3,2-c]pyridine-5(4*H*)-acetate). Clopidogrel has the following structure:

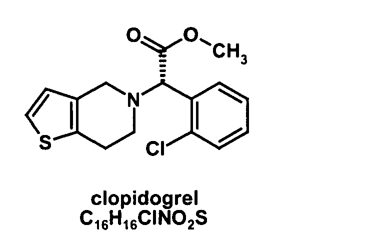

For the purpose of the specification, the term "clopidogrel" refers to the (S)-enantiomer, not to the racemate.

The platelet aggregation inhibiting activity of clopidogrel makes it an effective drug for reducing the incidence of ischemic strokes, heart attacks or claudication due to vascular diseases such as arteriosclerosis. By inhibiting platelet aggregation, clopidogrel reduces the chance of arterial blockage, thus preventing strokes and heart attacks. US 5,576,328 describes a method of preventing the occurrence of a secondary ischemic event by administration of clopidogrel, and is incorporated by reference herein.

Clopidogrel is more effective in blocking platelet aggregation than acetyl salicylic acid and is much gentler on the gastrointestinal tract. Clopidogrel is more effective than acetyl salicylic acid even at much lower dosage. A dosage of 75 mg of base equivalent has been shown to be more effective than a dosage of 325 mg of acetyl salicylic acid. In addition to being more effective, clopidogrel produces much less gastrointestinal bleeding than acetyl salicylic acid.

Clopidogrel may be administered e.g. as its hydrogensulfate salt. US 4,529,596, US 5,132,435, US 6,180,793, US 6,215,005 and US 6,258,961 disclose methods that can be used to prepare clopidogrel hydrogensulfate. Different polymorphs of clopidogrel hydrogensulfate have been disclosed in the prior art. WO 99/65915 discloses two polymorphs referred to as Forms I and II. Form I is also disclosed in EP 281 459.

The hydrobromide salt of clopidogrel has also been disclosed in the prior art.

In this regard it can be referred to US 4,847,265. The hydrobromide salt may be prepared by the action of the corresponding acid on the base in solution in a solvent from which it precipitates. In example 1 (f) of US 4,847,265 it is disclosed that 150 ml of an aqueous solution of sodium bicarbonate are added to a suspension of 20 g of the camphor-10-sulfonic acid salt of methyl-α-5(4,5,6,7-tetrahydro(3,2-c)thieno pyridyl) (2-chlorophenyl)-acetate (SR 25990 B) in 200 ml of CH₂Cl₂. The residue obtained after separation of the organic phase, drying and evaporation of the solvents is dissolved in 150 ml of diethyl or diisopropyl ether, and 4.4 ml of a 48% (wt/v) aqueous solution of hydrobromic acid are added drop-wise. The precipitate formed is isolated. After drying, 14.4 g of crystals are obtained with a melting point of 111°C. 13.4 g of these crystals are recrystallized from a mixture of isopropyl ether (100 ml) and isopropanol (150 ml) to give 10.2 g of analytically pure hydrobromide: m.p. = 140°C.

The method for the preparation of crystalline clopidogrel hydrobromide disclosed in US 4,847,265 has the disadvantage that the analytically pure hydrobromide is obtained by recrystallization from a solvent mixture containing isopropyl ether. However, isopropyl ether tends to spontaneously form explosive peroxides with oxygen from the atmosphere. The oxidation of isopropyl ether is facilitated, as there is a single hydrogen atom in α-position of the oxygen atom which can be homolytically decleaved, thereby forming a stabilized tertiary radical having a donor atom (oxygen) in α-position. The thus obtained radical may react with molecular oxygen from the atmosphere thereby forming a peroxy radical. Said peroxy radical may decleave a hydrogen radical from another isopropyl group forming a hydroperoxide. As such peroxides tend to explode spontaneously, particularly when the isopropyl ether is evaporated and the peroxides are concentrated, isopropyl ether is among the most dangerous solvents at all.

Several documents of the prior art mention clopidogrel hydrobromide and refer to US 4,847,265. However, these references do not disclose any preparation of clopidogrel hydrobromide differing from the method described in US 4,847,265. In this regard it can be referred to e.g. US 6,635,763, US 6,737,411, US-A 2004/24011 and US-A 2004/24012.

Polymorph forms of clopidogrel hydrobromide are not disclosed in any of these prior art documents.

Solid state physical properties of pharmaceutically active compounds include, for example, the flowability of the milled solid. Flowability affects the ease with which the material is handled during processing into a pharmaceutical product. When particles of the powdered compound do not flow past each other easily, a formulation specialist must take that fact into account when developing a tablet or capsule formulation, which may necessitate the use of glidants such as colloidal silicon dioxide, talc, starch or tribasic calcium phosphate.

The morphology of a solid substance usually influences its physical properties, such as thermodynamic stability, density, bulk density, compressibility, hardness, impact resistance, brittleness, elasticity, friction coefficient, shear modulus, torsion modulus, thermal stability (melting, boiling or sublimation point), hygroscopicity, adhesion to surfaces, electrostatic charging, electrical and thermal conductivity, dielectric constant, magnetic susceptibility, reflection and diffraction of irradiation, color, and the like. In certain cases the morphology may additionally cause an anisotropy of said properties.

Another important solid state property of a pharmaceutical compound is its rate of dissolution in aqueous fluid. The rate of dissolution of an active ingredient in a patient's stomach fluid can have therapeutic consequences because it imposes an upper limit on the rate at which an orally-administered active ingredient can reach the bloodstream. The rate of dissolution is also a consideration in formulating syrups, elixirs and other liquid medicaments. The solid state form of a compound may also affect its behavior on compaction and its storage stability. These properties may also be influenced by the interaction of the solid drug with other drugs and/or the excipients usually added as builder, binder, filler, surfactant, disintegrant, and the like.

These physical characteristics reflect the differences in the conformation and orientation of the molecules in the unit cell, which defines a particular polymorphic form of a substance.

The polymorphic form may give rise to thermal behavior different from that of the amorphous material or another polymorphic form. Thermal behavior is measured in the laboratory by such techniques as capillary melting point, thermogravimetric analysis (TGA) and differential scanning calorimetry (DSC), and can be used to distinguish some polymorphic forms from others. A particular polymorphic form may also give rise to distinct properties that may be detectable by powder X-ray diffraction, solid state NMR spectroscopy, Raman and infrared spectroscopy.

There is a strong need in pharmaceutical industry for active ingredients of uniform morphology. It is known that various polymorphs differ from each other significantly in their important properties (e.g. dissolution speed, bioavailability, interindividual variation, chemical stability). Also from a technological point of view there is a strong need for active agents with a well-defined morphology, because the working-up and processing properties of the various polymorphs (e.g. filtrability, drying, solubility, readiness to be compressed into tablets) can differ from each other significantly.

The discovery of new crystalline forms of a pharmaceutically useful compound provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic.

Thus, it is an object of the present invention to provide polymorphs of clopidogrel which exhibit advantageous physical properties, thereby improving the pharmacological properties of the drug and/or its processability when formulated to pharmaceutical dosage forms, such as tablets, pellets, granules, and powders. The polymorphs should have a uniform morphology meeting the above requirements and should allow manufacturing in a reproducible manner on an industrial scale. The skilled person should have the possibility to choose from different forms of clopidogrel with different physical properties. In particular, the present invention aims at providing clopidogrel in a therapeutically stable form which is particularly suitable for preparing medicaments.

It has been surprisingly found that one of the many salts of clopidogrel, namely clopidogrel hydrobromide, occurs in various polymorphs which differ in their physical properties. Five new crystalline anhydrous forms of clopidogrel hydrobromide, denoted as Forms A, B, C, D and E were found. Furthermore, it was unexpectedly found that clopidogrel also exists in several hydrate forms, and two hydrates, denoted as Forms HY-1 and HY-2, have been isolated by the inventors of the present invention. Of these hydrate forms, Form HY-1 is thermodynamically stable and provides significant technological advantages when formulated as a medicament. This form is most preferred according to the invention.

Unexpectedly, it was also found that the different polymorphic forms of clopidogrel hydrobromide can be obtained by crystallization from different solvents, however, the polymorph which is obtained can also depend on the purity of the starting material, in particular the optical purity. In particular, for obtaining polymorphic form HY-1 it may be necessary to carefully select the solvent for the recrystallization depending on the optical purity of the starting material.

In a first aspect the present invention provides five crystalline anhydrous modifications of clopidogrel hydrobromide, which are characterized by data selected from the group consisting of:
- a powder X-ray diffraction (PXRD) pattern with peaks at about 19.5, 25.4, 25.1, 23.0 and 20.9 degrees 2Θ,
- a differential scanning calorimetric thermogram having an endothermic peak at about 140°C and
- a FT Raman spectrum with peaks at about 3058, 2949 and 2931 cm⁻¹;
   said solid crystalline modification is denoted as "Form A"; while Form A may be characterized by any one of the above data, preferably Form A is characterized by all of the above data;
- a powder X-ray diffraction (PXRD) pattern with peaks at about 8.1, 8.8, 23.2, 24.6, 26.8, 22.8, and 16.6 degrees 20
- a differential scanning calorimetric thermogram having an endothermic peak maximum at about 141°C and
- a FT Raman spectrum with peaks at about 3063, 2999 and 2952 cm⁻¹;
   said solid crystalline modification is denoted as "Form B"; while Form B may be characterized by any one of the above data, preferably Form B is characterized by all of the above data;
- a powder X-ray diffraction (PXRD) pattern with peaks at about 6.1, 9.8 and 10.3 degrees 2Θ ,
- a differential scanning calorimetric thermogram having an endothermic peak maximum at about 139°C and
- a FT Raman spectrum with peaks at about 3089, 2984 and 2934 cm⁻¹;
   said solid crystalline modification is denoted as "Form C"; while Form C may be characterized by any one of the above data, preferably Form C is characterized by all of the above data;
- a powder X-ray diffraction (PXRD) pattern with peaks at about 9.0, 23.7, 24.9 and 14.4 degrees 2Θ ,
- a differential scanning calorimetric thermogram having an endothermic peak maximum at about 138.5°C and
- a FT Raman spectrum with peaks at about 3106, 3062, 2953 cm⁻¹;
   said solid crystalline modification is denoted as "Form D"; while Form D may be characterized by any one of the above data, preferably Form D is characterized by all of the above data; and
- a powder X-ray diffraction (PXRD) pattern with peaks at about 20.6, 23.0, 22.0 and 25.1 degrees 2Θ ,
- a differential scanning calorimetric thermogram having an endothermic peak maximum at about 146 °C and
- a FT Raman spectrum with peaks at about 3109, 3064, 2955 cm⁻¹;
   said solid crystalline modification is denoted as "Form E"; while Form E may be characterized by any one of the above data, preferably Form E is characterized by all of the above data.

In a second aspect the present invention provides a hydrate of crystalline clopidogrel hydrobromide. Preferably, the hydrate is a monohydrate.

In a preferred embodiment the present invention provides two crystalline hydrates of clopidogrel hydrobromide, which are characterized by data selected from the group consisting of:
- a powder X-ray diffraction (PXRD) pattern with peaks at about 12.5, 20.0, 21.9, 27.9 and 28.4 degrees 2Θ,
- a differential scanning calorimetric thermogram having an endothermic peak maximum at about 110°C and
- a FT Raman spectrum with peaks at about 3074, 3043, 3001, and 2938 cm⁻¹;
   said solid crystalline modification is denoted as "Form HY-1"; while Form HY-1 may be characterized by any one of the above data, preferably Form HY-1 is characterized by all of the above data; and
- a powder X-ray diffraction (PXRD) pattern with peaks at about 26.5, 7.5, 22.8, 17.4 and 10.3 degrees 2Θ,
- a differential scanning calorimetric thermogram having an endothermic peak maximum at about 110 °C and
- a FT Raman spectrum with peaks at about 3059, 2973 and 2922 cm⁻¹;
   said solid crystalline modification is denoted as "Form HY-2", while Form HY-2 may be characterized by any one of the above data, preferably Form HY-2 is characterized by all of the above data.

The powder X-ray diffraction patterns were measured with a Siemens D5000 apparatus, the DSC measurements were made with a Perkin Elmer DSC 7 and the FT Raman spectra were recorded with a Bruker RFS 100 spectrometer. Details of the measurements are explained at the end of the description under the heading methods and devices.

The inventors of the present invention have found the above crystal modifications of clopidogrel hydrobromide, i.e. seven new polymorphs, namely 2 hydrates and 5 anhydrous forms.

The crystalline forms of clopidogrel hydrobromide according to the invention may be obtained by different crystallization methods:
- crystallization from hot saturated solutions;
- precipitation from a warm or cold solution by adding an antisolvent;
- slow evaporation of the solvent from solutions at room temperature or below;
- lyophilization; or
- stirring of a suspension of a form or mixtures of different forms in a solvent at a constant temperature or a temperature range.

All forms were characterised by DSC, TGA, PXRD and FT-Raman spectroscopy. Additionally, forms A, B and HY-1 were characterized by single crystal X-ray diffractometry.

In a further aspect, the present invention provides a process for preparing clopidogrel hydrobromide having at least one of the characteristics of any one of Form A, Form B, Form C, Form D, Form E, or a hydrated form, e.g. Form HY-1 or Form HY-2, (such as the PXRD peaks and/or FT Raman peaks and/or DSC peaks disclosed herein) comprising the steps of
(i) providing a solution of clopidogrel hydrobromide in a solvent; and
(ii) precipitating clopidogrel hydrobromide therefrom.

Preferably, the solvent does not contain isopropyl ether. The wet sample is preferably dried.

Preferably, the solvent is selected from the group consisting of water, aliphatic alcohols, aliphatic nitrils, aliphatic esters, aliphatic or aromatic hydrocarbons, aliphatic halogenated hydrocarbons, aliphatic ethers and mixtures thereof.

More preferably, the solvent is selected from the group consisting of water, methanol, ethanol, n-propanol, n-butanol, acetonitrile, ethylacetate, toluene, hexane, dichloromethane, chloroform, tetrachloromethane, tetrahydrofurane, diethylether, and mixtures thereof.

Most preferably, the solvent is selected from the group consisting of water, n-propanol/water, n-butanol/water, n-butanol, acetonitrile, toluene, tetrahydrofurane, ethylacetate, n-butanol/hexane, ethylacetate/methanol, chloroform/ethylacetate, toluene/methanol, tetrachloromethane/dichloromethane, n-butanol/diethylether, chloroform, and ethanol.

In a preferred embodiment of the process according to the invention step (ii) is initiated by evaporation and/or cooling of the solvent.

In another preferred embodiment of the process according to the invention step (i) is performed by means of a solvent capable of dissolving clopidogrel hydrobromide and step (ii) is initiated by adding an antisolvent. Preferably, the solvent is selected from the group consisting of methanol, ethanol, n-propanol, i-propanol, n-butanol, n-pentanol, acetic acid, dichloromethane, chloroform, acetone, acetonitrile, nitromethane, dioxane, tetrahydrofurane, ethylacetate, toluene, benzene, xylene, dimethylformamide and dimethylsulfoxide and the antisolvent is selected from the group consisting of tetrachloromethane, petrolether, diethyl ether, n-hexane, cyclohexane and n-decane.

For the purpose of the specification a "solvent" is preferably defined as a compound or mixture of compounds in 100 ml of which at 25°C clopidogrel hydrobromide is soluble in an amount of more than 1 g, more preferably more than 2 g, still more preferably more than 5 g, most preferably more than 7.5 g and in particular more than 10 g.

For the purpose of the specification an "antisolvent" is preferably defined as a compound or mixture of compounds in 100 ml of which at 25°C clopidogrel hydrobromide is soluble in an amount of less than 1 g, more preferably less than 0.5 g, still more preferably less than 0.1 g, most preferably less than 0.05 g and in particular less than 0.01 g.

Which solvents, antisolvents and crystallization conditions can be used to obtain each of the different polymorphic forms of clopidogrel hydrobromide can be seen from the examples.

The present invention also provides pharmaceutical compositions comprising the modifications of clopidogrel hydrobromide according to the invention and their use for the manufacture of a medicament for inhibiting platelet aggregation. Particularly preferred are pharmaceutical compositions containing the polymorph form HY-1 of clopidogrel hydrobromide. Preferred are also pharmaceutical compositions in which at least 90%, more preferably at least 95%, more preferably 99% or 100% of the clopidogrel hydrobromide is of one well-defined polymorphic form, most preferred of polymorphic form HY-1. Form HY-1 is the thermodynamically most stable polymorphic form of clopidogrel hydrobromide.

As a platelet inhibitor, clopidogrel is effective at suppressing the lethal effects of blood clotting. Platelet aggregation often occurs around damaged blood vessels. The blood vessels may only have minor fissures or plaques to induce platelet aggregation.

Platelet aggregation leads to the blockage of arteries, thus increasing the risk of primary and secondary strokes and heart attacks. By inhibiting platelet aggregation, clopidogrel hydrobromide reduces the risk of heart attacks and strokes. Clopidogrel is particularly effective in the secondary prevention of ischemic events, which are defined in the art as a decrease in the blood supply to a bodily organ, tissue, or part caused by constriction or obstruction of the blood vessels.

Pharmaceutical compositions of the present invention contain clopidogrel hydrobromide Forms A, B, C, D, E and the hydrates HY-1 and HY-2 optionally in mixture with other Form(s) or amorphous clopidogrel and/or active ingredients. The clopidogrel hydrobromides according to the present invention are ideal for pharmaceutical compositions in that they have a purity of at least about 90 wt.-%, more preferably at least about 95 wt.-%, and most preferably at least about 99 wt.-% (area percentage as measured by HPLC). In addition to the active ingredient(s), the pharmaceutical compositions of the present invention may contain one or more excipients. Excipients are added to the composition for a variety of purposes.

Preferably, each polymorph form of clopidogrel hydrobromide of the present invention is substantially free of all other polymorph forms and anhydrous forms of clopidogrel hydrobromide, and preferably each of the polymorph forms of clopidogrel hydrobromide of the present invention comprises not more than 20%, more preferably not more than 10%, in particular not more than 5%, such as not more than 2%, or not more than 1% of any other polymorphic or amorphous form of clopidogrel hydrobromide or is free of any such other form.

Diluents increase the bulk of a solid pharmaceutical composition, and may make a pharmaceutical dosage form containing the composition easier for the patient and care giver/physician to handle. Diluents for solid compositions include, for example, microcrystalline cellulose (e.g. Avicel®), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit®), potassium chloride, powdered cellulose, sodium chloride, sorbitol and talc.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e. g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel®), hydroxypropyl methyl cellulose (e.g. Methocel®), liquid glucose, magnesium aluminum silicate, maltodextrin, methylcellulose, polymethacrylates, povidone (e. g. Kollidon, Plasdone®), pregelatinized starch, sodium alginate and starch.

The dissolution rate of a compacted solid pharmaceutical composition in the patient's gastrointestinal tract may be increased by the addition of a disintegrant to the composition. Disintegrants include alginic acid, carboxymethylcellulose calcium, carboxymethylcellulose sodium (e.g. Ac-Di-Sol, Primellose®), colloidal silicon dioxide, croscarmellose sodium, crospovidone (e.g. Kollidon®, Polyplasdone®), guar gum, magnesium aluminum silicate, methyl cellulose, microcrystalline cellulose, polacrilin potassium, powdered cellulose, pregelatinized starch, sodium alginate, sodium starch glycolate (e.g. Explotab®) and starch.

Glidants can be added to improve the flowability of a non-compacted solid composition and to improve the accuracy of dosing. Excipients that may function as glidants include colloidal silicon dioxide, magnesiumtrisilicate, powdered cellulose, starch, talc and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc and zinc stearate.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol and tartaric acid.

Solid and liquid compositions may also be dyed using any pharmaceutically acceptable colorant to improve their appearance and/or facilitate patient identification of the product and unit dosage level.

In liquid pharmaceutical compositions of the present invention, clopidogrel hydrobromide and any other solid excipients are dissolved or suspended in a liquid carrier such as water, vegetable oil, alcohol, polyethylene glycol, propylene glycol or glycerin.

Liquid pharmaceutical compositions may contain emulsifying agents to disperse uniformly throughout the composition an active ingredient or other excipient that is not soluble in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol and cetyl alcohol.

Liquid pharmaceutical compositions of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth and xanthan gum.

Sweetening agents such as sorbitol, saccharin, sodium saccharin, sucrose, aspartame, fructose, mannitol and invert sugar may be added to improve the taste.

Preservatives and chelating agents such as alcohol, sodium benzoate, butylated hydroxy toluene, butylated hydroxyanisole and ethylenediamine tetraacetic acid may be added at levels safe for ingestion to improve storage stability.

According to the present invention, a liquid composition may also contain a buffer such as gluconic acid, lactic acid, citric acid or acetic acid, sodium gluconate, sodium lactate, sodium citrate or sodium acetate.

It is understood that the crystal form of clopidogrel hydrobromide is destroyed in liquid compositions comprising a solution of clopidogrel hydrobromide and such compositions are not part of the present invention. However, the use of polymorphic clopidogrel hydrobromide according to the present invention for the preparation of such liquid compositions is covered by the present invention as well as liquid pharmaceutical compositions as such, which comprise polymorphic clopidogrel as a solid, such as suspensions.

Selection of excipients and the amounts used may be readily determined by the formulation scientist based upon experience and consideration of standard procedures and reference works in the field.

The solid compositions of the present invention include powders, granulates, aggregates and compacted compositions. The dosages include dosages suitable for oral, buccal, rectal, parenteral (including subcutaneous, intramuscular, and intravenous), inhalant and ophthalmic administration. Although the most suitable administration in any given case will depend on the nature and severity of the condition being treated, the most preferred route of the present invention is oral. The dosages may be conveniently presented in unit dosage form and prepared by any of the methods well-known in the pharmaceutical arts.

Dosage forms include solid dosage forms like tablets, powders, capsules, suppositories, sachets, troches and losenges, as well as liquid syrups, suspensions and elixirs.

The dosage form of the present invention may be a capsule containing the composition, preferably a powdered, granulated or pelleted solid composition of the invention, within either a hard or soft shell. The shell may be made from gelatin and optionally contain a plasticizer such as glycerin and sorbitol, and an opacifying agent or colorant.

The active ingredient and excipients may be formulated into compositions and dosage forms according to methods known in the art.

A composition for tableting or capsule filling may be prepared by wet granulation. In wet granulation, some or all of the active ingredients and excipients in powder form are blended and then further mixed in the presence of a liquid, typically water, that causes the powders to clump into granules. The granulate is screened and/or milled, dried and then screened and/or milled to the desired particle size. The granulate may then be tableted, or other excipients may be added prior to tableting, such as a glidant and/or a lubricant.

A tableting composition may be prepared conventionally by dry blending. For example, the blended composition of the actives and excipients may be compacted into a slug or a sheet and then comminuted into compacted granules. The compacted granules may subsequently be compressed into a tablet.

As an alternative to dry granulation, a blended composition may be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention may comprise any of the aforementioned blends and granulates that were described with reference to tableting, however, they are not subjected to a final tableting step.

Capsules, tablets and lozenges, and other unit dosage forms preferably contain a base equivalent of about 75 mg clopidogrel hydrobromide Forms A, B, C, D or the hydrates HY-1 or HY-2. The unit dosage form as used herein refers to the amount of the various forms of clopidogrel contained in the vehicle of administration, such as a tablet or a capsule. In a preferred embodiment, the unit dosage in a tablet for oral administration contains a base equivalent of about 25 mg to 150 mg. Most preferably, it is about 75 mg base equivalent. One skilled in the art would appreciate that other unit dosages may be made as necessary in a routine fashion.
Fig. 1 shows the DSC- and TGA-curve of Form HY-1 obtained in example 5d).
Fig. 2 shows the DSC- and TGA-curve of Form HY-2 obtained in example 6.
Fig. 3 shows the DSC- and TGA-curve of Form A obtained in example 1a).
Fig. 4 shows the DSC- and TGA-curve of Form B crystallized from 1:4 n-butanol/diethyl ether mixture according to example 2a).
Fig. 5 shows the DSC- and TGA-curve of Form C obtained in example 3.
Fig. 6 shows the DSC- and TGA- curve of anhydrous Form D obtained in example 4.
Fig. 7 shows the powder X-ray diffraction pattern of Form HY-1, obtained in example 5d).
Fig. 8 shows the powder X-ray diffraction pattern of Form A, obtained in example 1a).
Fig. 9 shows the powder X-ray diffraction pattern of Form B, obtained in example 2a).
Fig. 10 shows the powder X-ray diffraction pattern of Form C, obtained in example 3.
Fig. 11 shows the powder X-ray diffraction pattern of Form D, obtained in example 4.
Fig. 12 shows the powder X-ray diffraction pattern of Form HY-2, obtained in example 6.
Fig. 13 shows the Raman spectrum of Form HY-1, obtained in example 5d).
Fig. 14 shows the Raman spectrum of Form HY-2, obtained in example 6.
Fig. 15 shows the Raman spectrum of Form A, obtained in example 5d).
Fig. 16 shows the Raman spectrum of Form B, obtained in example 2a).
Fig. 17 shows the Raman spectrum of Form C, obtained in example 3.
Fig. 18 shows the Raman spectrum of Form D, obtained in example 4.
Fig. 19 shows an Ortep diagram (top) and molecular diagram (bottom) of the molecules in the Form HY-1. Note the chiral centers (C1, N8) are S and R, respectively.

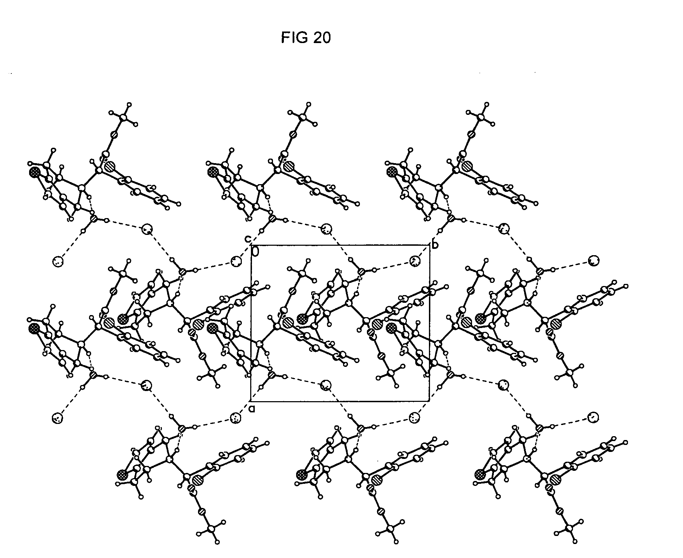

Fig. 20 shows the packing diagram of the Form HY-1 indicating the alternate helical chains of O-H···Br interactions in head-tail fashion (···[H₂O]···[BR]-···[H₂O]···[BR]-···). [CG]+ is acting as spacer between these chains by forming N-H···O hydrogen bonds to water molecules.
Fig. 21 shows an Ortep diagram (top) and molecular diagram (bottom) of the molecules in the Form A structure. Note the chiral centers (C1, N8, C19, N26) in both molecules are 'S' type.

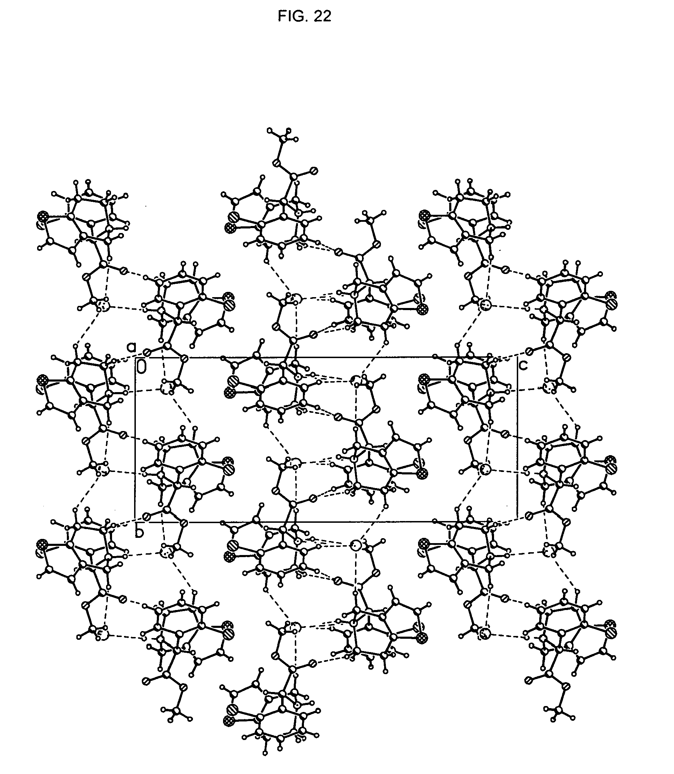

Fig. 22 shows the crystal structure of Form A. The two independent molecules are separately forming C-H···Br hydrogen bonded chains along the *b*-axis in head-tail fashion ([CG]+···[BR]-···[CG]+···[BR]-···). These chains are further connected by N-H···Br interactions along the c-axis forming columns parallel to the *b*-axis.
Fig. 23 shows the Ortep diagram (top) and molecular diagram (bottom) of the molecules in the Form B structure. Note the chiral centers (C1, N8) and (C19, N26) in both molecules are (S, R) type.

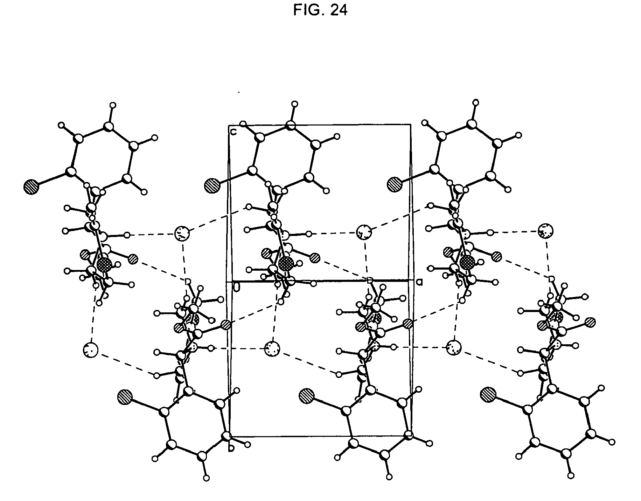

Fig. 24 shows the crystal structure of Form B indicating a network of N-H···Br, C-H···O and C-H···Br hydrogen bonds in head-tail fashion ([CG]+··· [BR]-··· [CG]+··· [BR]-···) forming a columnar structure with bromine atoms filling the interstitial space.
Fig. 25 shows the DSC- and TGA-curve of Form E, obtained in example 8.
Fig. 26 shows the powder X-ray diffraction pattern of Form E, obtained in example 8.
Fig. 27 shows the Raman spectrum of Form E, obtained in example 8.

The following examples further illustrate the present invention but are not to be construed as limiting its scope:

### Example A

126 g (0,3 mol) optically purified S(+) clopidogrel hydrogensulphate are mixed with 500 ml of dichloromethane and 720 ml of saturated sodium hydrogencarbonate (gradual addition is necessary to avoid foaming). The mixture is agitated at 15°C until clarification (approx. 20 minutes) and then the layers are separated in a separatory funnel. The organic layer is dried over anhydrous sodium sulphate. On evaporation of the solvent clopidogrel base is obtained as a colorless oil in quantitative yield (96,5 g).

The clopidogrel base was dissolved in ethylactate, and 48% hydrobromic acid was added dropwise (during approx. 30 minutes) under cooling to 12 - 15°C on water bath. The formed suspension was stirred for additional 30 minutes, then heated under condenser to the boiling point of the solvent and let slowly cool down under continuous stirring. Then it was cooled down to 10°C, filtered and washed with 275 ml of ethylacetate. The filter cake was then sucked dry. The product was then dried in vaccuum drier for 4 hours at 50°C and 22 torr and then for 3 hours at the same temperature and 5 torr. Finally the crystalline product was homogenized by sieving through 73 mesh sieve and adjusted. The yield was 520 g (98%).
a) The clopidogrel hydrogensulphate which was used in the process had an optical purity of 99.5% (S)-enantiomer to 0.5% (R)-enantiomer. The optical purity of the clopidogrel base obtained as an intermediate product was the same. The obtained clopidogrel hydrobromide was a mixture of different hydrate and anhydrate forms with no well-defined polymorphic structure.
b) The clopidogrel hydrogensulphate which was used in the process had an optical purity of 98% (S)-enantiomer and 2% (R)-enantiomer. The optical purity of the clopidogrel base obtained as an intermediate product was the same. The obtained clopidogrel hydrobromide was clopidogrel hydrobromide of the pure polymorphic form HY-1 as established by PXRD, Raman spectroscopy and DSC measurements.

### Example B

Example A was repeated but instead of ethylacetate a 1:1 mixture of ethylacetate and isopropylacetate was used.
a) The clopidogrel hydrogensulphate which was used in the process had an optical purity of 99.5% (S)-enantiomer to 0.5% (R)-enantiomer. The optical purity of the clopidogrel base obtained as an intermediate product was the same. The obtained clopidogrel hydrobromide was pure polymorphic Form HY-1 as established by PXRD, Raman spectroscopy and DSC measurements.
b) The clopidogrel hydrogensulphate which was used in the process had an optical purity of 98% (S)-enantiomer to 2% (R)-enantiomer. The optical purity of the clopidogrel base obtained as an intermediate product was the same. The obtained clopidogrel hydrobromide was pure polymorphic Form HY-1 as established by PXRD, Raman spectroscopy and DSC measurements.

In the following preparation examples clopidogrel hydrobromide as obtained in example Ba) has been used to obtain Forms A, B, C, D, E and HY-2 and to exemplify further methods for obtaining Form HY-1. These forms are then used to prepare other polymorphic forms of clopidogrel hydrobromide.

### Example 1: Preparation of polymorph Form A of clopidogrel hydrobromide

a) 100mg of clopidogrel hydrobromide HY-1 were dissolved in 10 ml of toluene at 100°C and allowed to stir at room temperature for 3-5 hours. After 5 hours the crystals were filtered off.
b) Alternatively, Form A was obtained by adding 1 ml of methanol to a saturated solution of 100 mg clopidogrel hydrobromide HY-1 in 10ml ethylacetate at a temperature of 70°C and allowing the solvent to evaporate at room temperature. After 24 hours the crystals were filtered off.
c) Form A was also obtained by slow evaporation of a saturated solution of 100 mg clopidogrel hydrobromide HY-1 in 5ml n-propanol, 5ml *n*-butanol, 10 ml n-butanol/hexane 5:5 (v/v), 10 ml chloroform/ethylacetate 3:7 (v/v), 10 ml toluene/methanol 9:1 (v/v), and 10 ml tetrachloromethane/dichloromethane 7:3 (v/v), respectively, and allowing the solvents at a temperature of 25°C to evaporate for 24 hours at ambient pressure. After 24 hours the crystals were filtered off.

### Example 2: Preparation of polymorph Form B of clopidogrel hydrobromide

a) 100 mg of clopidogrel hydrobromide HY-1 were dissolved in 3-5 ml n-butanol (by warming to a temperature of 100°C) and 8-10 ml of diethylether were added slowly over 3-5 minutes until a saturated solution was obtained. Evaporation of the solvent at room temperature yielded Form B as a precipitate.
b) Under identical conditions the solvent mixtures toluene/ethylacetate 5:5 (v/v) and chloroform/toluene 3:7 (v/v) yielded mixtures of Forms A and B.

### Example 3: Preparation of polymorph Form C of clopidogrel hydrobromide

Form C was observed by evaporating the solvent at a temperature of 25°C at ambient pressure from a saturated solution of 100 mg clopidogrel hydrobromide HY-1 in 5 ml of chloroform for 24 hours.

### Example 4: Preparation of polymorph Form D of clopidogrel hydrobromide

Form D was observed by evaporating the solvent at a temperature of 25°C at ambient pressure from a saturated solution of 100 mg clopidogrel hydrobromide HY-1 in 5 ml ethanol for 24 hours.

### Example 5: Preparation of polymorph Form HY-1 of clopidogrel hydrobromide

a) This hydrated form can be obtained by the precipitation method by adding an antisolvent to a warm or cold solution of clopidogrel hydrobromide HY-1 in respective solvent.
b) Alternatively, Form HY-1 was obtained by stirring a super saturated solution of 200 mg clopidogrel hydrobromide any anhydrous form in 10 ml ethylacetate at room temperature for 3 hrs.
c) Form HY-1 was obtained by crystallising it from 1:1 mixtures of n-butanol/H₂O and n-propanol/H₂O. For that purpose, a solution of 300 mg clopidogrel hydrobromide HY-1 in 10ml n-butanol/H₂O 1:1 (v/v) and 10 ml n-propanol/H₂O 1:1 (v/v), respectively, was prepared and the solvents were allowed to evaporate at a temperature of 25°C for 24 hours ambient pressure. After 24-48 hours the crystals were filtered off.

### Example 6: Preparation of polymorph Form HY-2 of clopidogrel hydrobromide

This hydrated form was obtained by stirring a 1:1 mixture of 200 mg of the forms Form A and Form B in 3-5 ml toluene between 10-30°C for 1-4 days.

### Example 7:

Using the solvents/solvent mixtures and method examples specified in Tables 1 and 2, the different modifications of clopidogrel hydrobromide were obtained.

**Table 1: crystallization results from pure solvents**

| solvent | solubility | remarks | method example | Form |
|---|---|---|---|---|
| water | sparingly soluble in hot | needle like crystals | 1 | HY-1 |
| methanol | highly soluble | oily mass | 1 | no crystals |
| ethanol | highly soluble | solid mass | 1 | D |
| n-propanol | highly soluble | colourless crystals | 1 | Hy-1 |
| i-propanol | highly soluble | oily mass | 1 | no crystals |
| n-butanol | highly soluble | rod shaped crystals | 1 | A |
| n-pentanol | soluble | oily mass | 1 | no crystals |
| acetic acid | highly soluble | oily mass | 1 | no crystals |
| dichloromethane | highly soluble | oily mass | 1 | no crystals |
| chloroform | highly soluble | solid mass | 1 | C |
| tetrachloromethane | insoluble | ---- | 1 | ---- |
| acetone | soluble | oily mass | 1 | no crystals |
| acetonitrile | soluble | precipitated as solid | 1 | A |
| nitromethane | soluble | oily mass | 1 | no crystals |
| dioxane | soluble in hot | oily mass | 1 | no crystals |
| tetrahydrofurane | soluble in hot | crystals | 1 | A |
| ethylacetate | soluble in hot | crystals | 1 | A |
| toluene | soluble in hot | colourless crystals | 1 | A |
| benzene | sparingly soluble | ---- | 1 | no crystals |
| xylene | soluble in hot | ---- | 1 | no crystals |
| petrolether | insoluble | ---- | 1 | ---- |
| diethylether | insoluble | ---- | 1 | ---- |
| n-hexane | insoluble | ---- | 1 | ---- |
| cyclohexane | insoluble | ---- | 1 | ---- |
| n-decane | insoluble | ---- | 1 | ---- |
| dimethylformamide | soluble | oily mass | 1 | no crystals |

Method example 1: 100 mg of clopidogrel hydrobromide HY-1 obtained according to example Ba) was dissolved in 5 ml of the solvent on a water bath at 80-100°C with constant shaking and the solvent was allowed to evaporate slowly at room temperature for 24 hours at ambient pressure.

**Table 2: crystallization results from solvent mixtures**

| "first solvent"/ "second solvent" | Remarks | solvent ratio | method example | Form |
|---|---|---|---|---|
| n-propanol/ water | dissolving the substance in n-propanol (by slightly warming) and adding few drops of water | 1:1 | a | HY-1 |
| n-butanol/ water | dissolving the substance in n-butanol (by slightly warming) and adding few drops of water | 1:1 | a | HY-1 |
| n-butanol/ hexane | dissolving the substance in n-butanol and reducing the polarity by adding hexane until it becomes a saturated solution and allowing it to slowly evaporate at room temperature | 1:3 | b | A |
| ethylacetate/ methanol | adding few drops of methanol to the supersaturated solution of ethylacetate and allowing to evaporate at room temperature | 9:1 (v/v) | c | A |
| chloroform/ ethylacetate | dissolving the substance in chloroform and make a super saturated solution with ethylacetate and allowing it to slowly evaporate at room temperature | 1:3 | d | A |
| toluene/ methanol | adding few drops of methanol to supersaturated solution of toluene and allowing it to stand at room temperature | 9:1 | e | A |
| benzene/ acetonitrile | Decomposition | 1:1 | b | --- |
| tetrachloromethane/ dichloromethane | precipitation in solvent mixture, tetrachloromethane/ dichloromethane | 3:1 | a | A |
| dioxane/ether | forming an oily mass at the bottom | 1:3 | b | --- |
| n-butanol/diethyl ether | dissolving the substance in n-butanol (by warming slightly) and slowly adding the diethylether until it becomes a saturated solution and allowing it to evaporate at room temperature | 1:5 | f | B |

Method example a: 300 mg of clopidogrel hydrobromide HY-1 were dissolved in 10 ml of 1.1 solvent mixtures by warming to a temperature of 70°C followed by the evaporation of the solvent at room temperature for 24 hours. The precipitate was filtered off.

Method example b: 100 mg of clopidogrel hydrobromide HY-1 were dissolved in 3-5 ml of the first solvent at a temperature of 70°C. 8-10 ml of the second solvent were added to the solution over 3-5 minutes to reduce the polarity until it becomes a saturated solution. The solvent mixture was allowed to evaporate for 24 hours at room temperature at ambient pressure for 24 hours. The precipitate was filtered off.

Method example c: 100 mg of clopidogrel hydrobromide HY-1 were dissolved in 9 ml of the first solvent at a temperature of 70°C. 1ml of the second solvent were added to the supersaturated solution over 3-5 minutes. The solvent mixture was allowed to evaporate for 24 hours at room temperature at ambient pressure for 24 hours. The precipitate was filtered off.

Method example d: 100 mg of clopidogrel hydrobromide HY-1 were dissolved in 3-5 ml of the first solvent at a temperature of 50°C. 8-10 ml of the second solvent were added to provide a supersaturated solution. The solvent mixture was allowed to evaporate for 24 hours at room temperature at ambient pressure for 24 hours. The precipitate was filtered off.

Method example e: 100 mg of clopidogrel hydrobromide HY-1 were dissolved in 7-10 ml of the first solvent at a temperature of 90-100°C. 0.5-1 ml of the second solvent were added to the supersaturated solution over 1-2 minutes. The solvent mixture was allowed to stand for 24 hours at room temperature. The precipitate was filtered off.

Method example f: 100 mg of clopidogrel hydrobromide HY-1 were dissolved in 2-4 ml of the first solvent at a temperature of 70°C. 8-10 ml of the second solvent were added to the supersaturated solution over 3-5 minutes. The solvent mixture was allowed to stand for 24 hours at room temperature. The precipitate was filtered off.

### Example 8: Preparation of polymorph Form E of clopidogrel hydrobromide

Polymorph Form B of clopidogrel hydrobromide obtained in example 2a) was stored at room temperature in a closed glass container. After 8 to 10 month the sample was investigated by PXRD, DSC and Raman spectroscopy, and polymorphic Form E of clopidogrel hydrobromide was identified.

### Example 9: Preparation of the amorphous form of clopidogrel hydrobromide

The amorphous form was obtained by lyophilization of a 2% solution of clopidogrel hydrobromide HY1 in H2O/MeOH (1:1) which was frozen with liquid Nitrogen.

### Example 10: Stability of the forms (solvent mediated transformation studies)

To assess the stability of the anhydrous Forms A and B, solvent mediated transformation experiments were performed. This showed that Forms A and B are readily converted to the hydrate, i.e. HY-1 within a time span of 1 hr in apolar solvents containing minimal amounts of water. This was tested by viewing a few crystals of Form A/Form B under the microscope by placing them on a glass plate with a cover-slip, adding a drop of water and spreading around the cover. Needle like crystals were formed within a time span of 30 min which corresponded to HY-1. Stirring a 1:1 mixture of Forms A and B in toluene (200 mg, in 3-5 ml) between 10-30°C for 1-4 days produced polymorph HY-2 which was formed after 1 day. The reaction mixture was checked after 4 days and again later 2-4 weeks by measuring PXRD, DSC, TGA, Raman and it was confirmed as form HY-2.

**Table 3: Results of the solvent mediated transformation studies**

| Mixture | Solvent | Time/temp/remarks | Resulting form |
|---|---|---|---|
| A:B (1:1) | water | 1 day/20°C | HY-1 |
| A:B (1:1) | water | 1 day/20°C | HY-1 |
| A or B | water | 30 min/25°C | HY-1 |
| A:B (1:1) | toluene | 1 day/10-30°C | HY-2 |

### Example 11: Thermal Analysis

### DSC and TGA Analysis

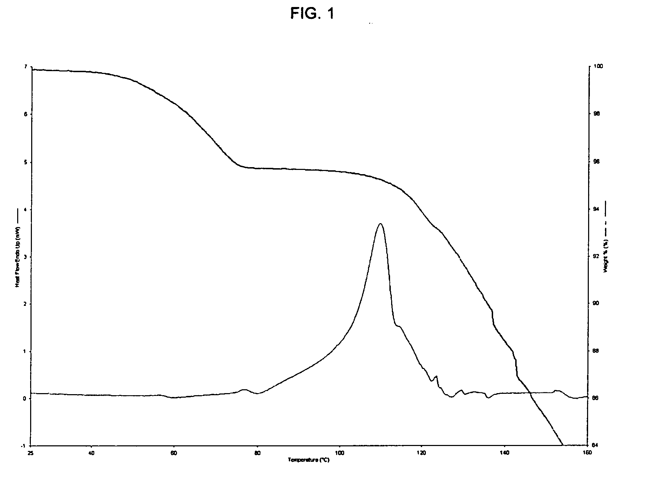

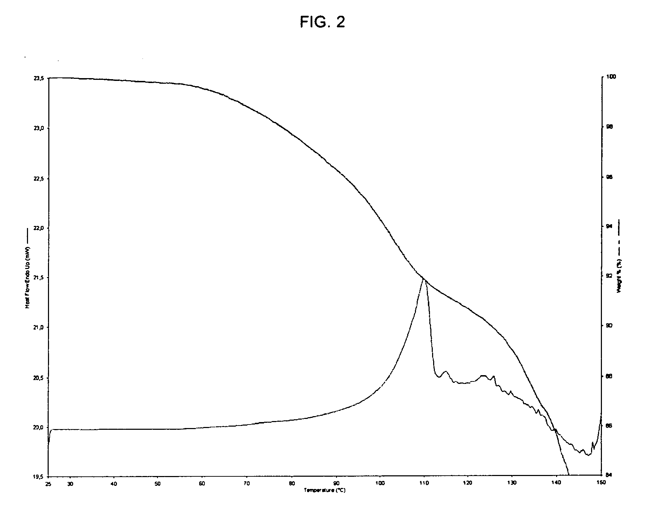

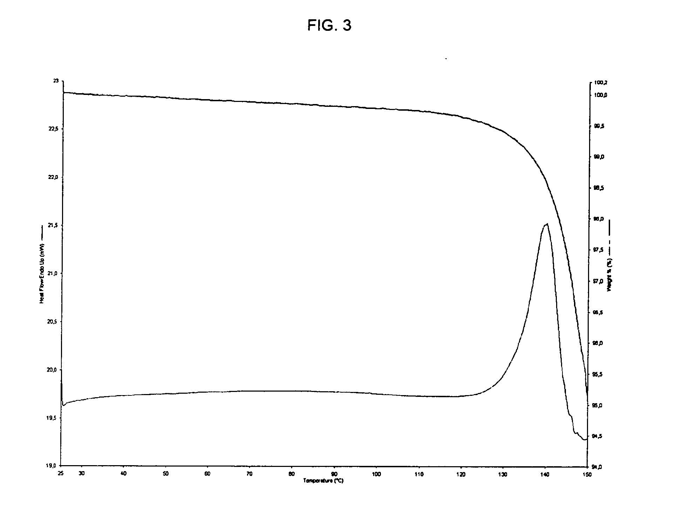

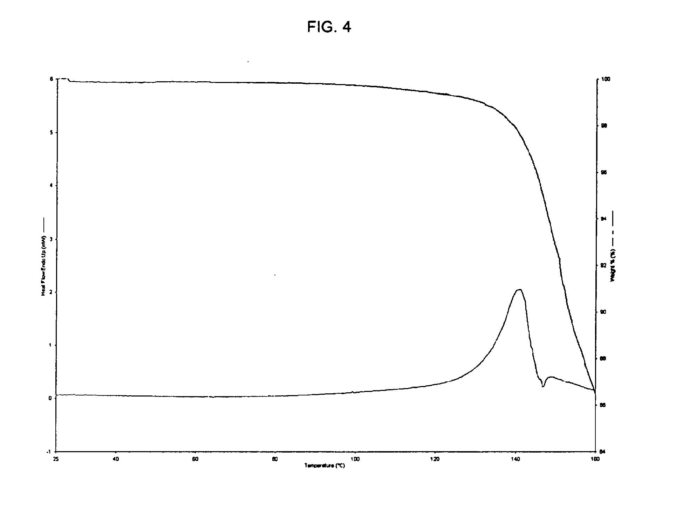

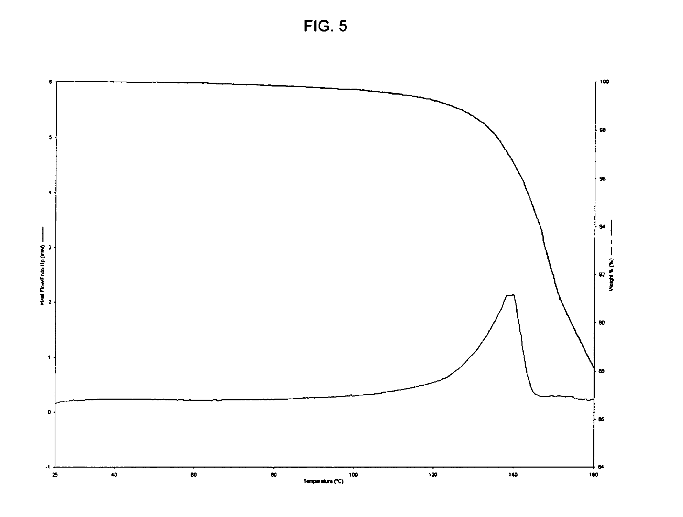

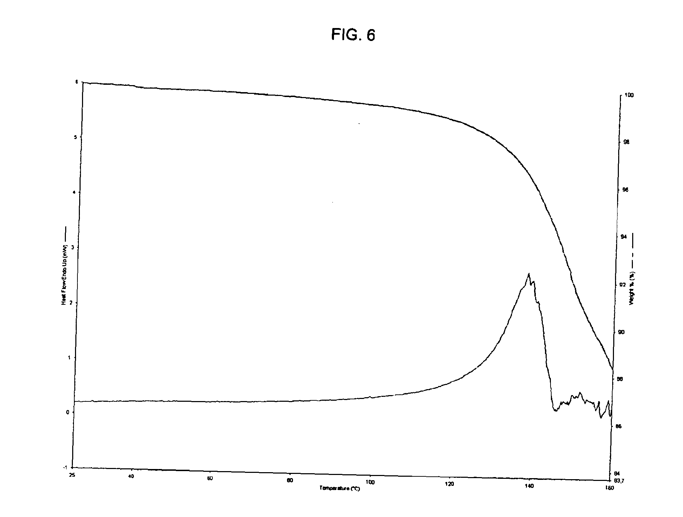

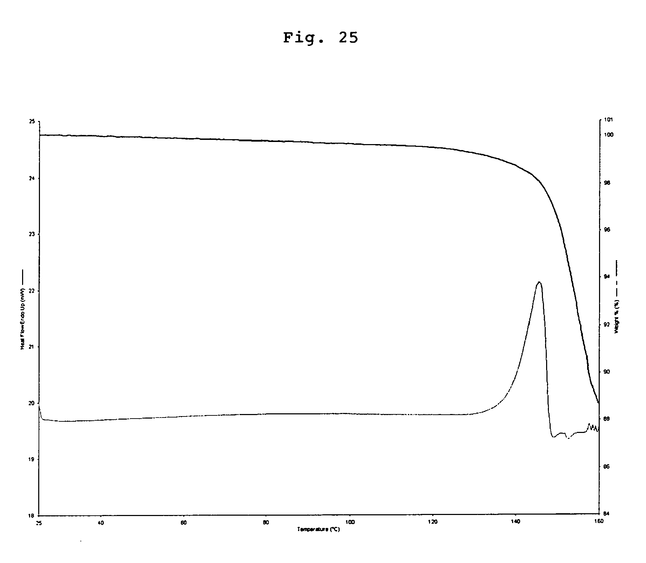

Diffrential scanning calorimetry (DSC) thermograms and thermogravimetric analysis (TGA) for the hydrates HY-1 and HY-2 and the anhydrous modifications A to E of clopidogrel hydrobromide were recorded on a Perkin Elmer DSC-7 and Perkin Elmer TGA-7. The thermograms are shown in Figures 1 to 6 and 25.

### Example 12: Powder X-Ray Diffraction

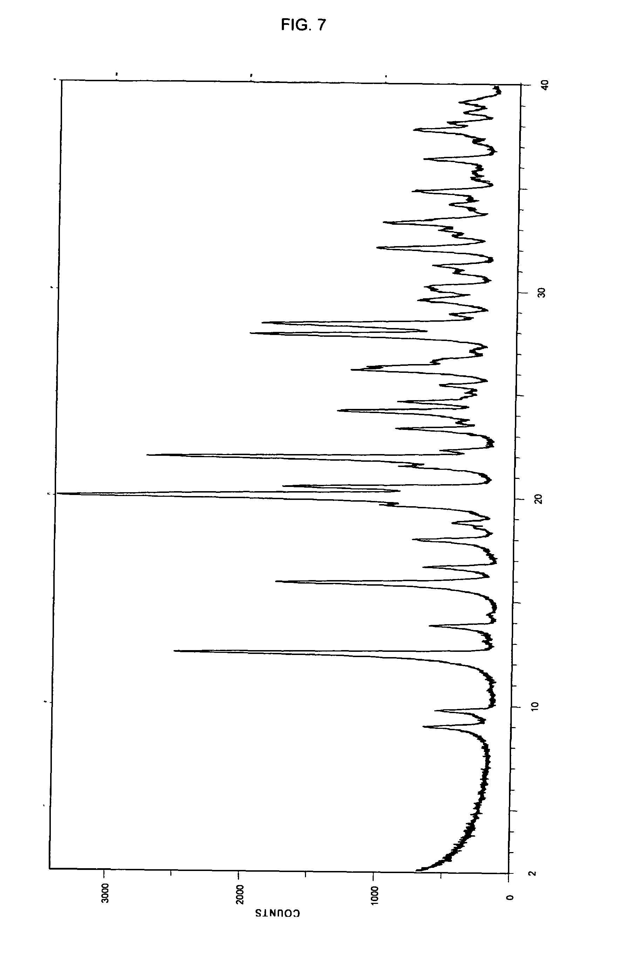

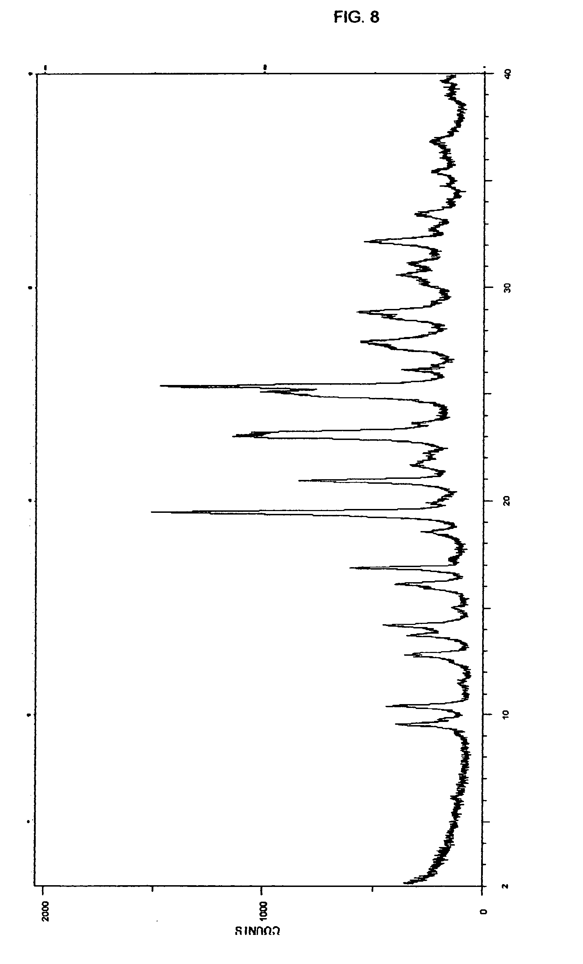

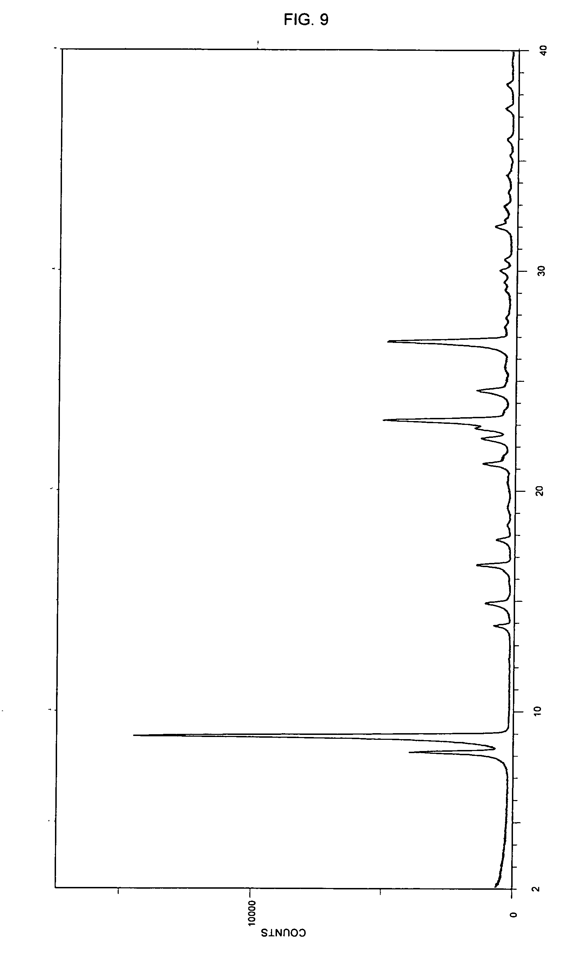

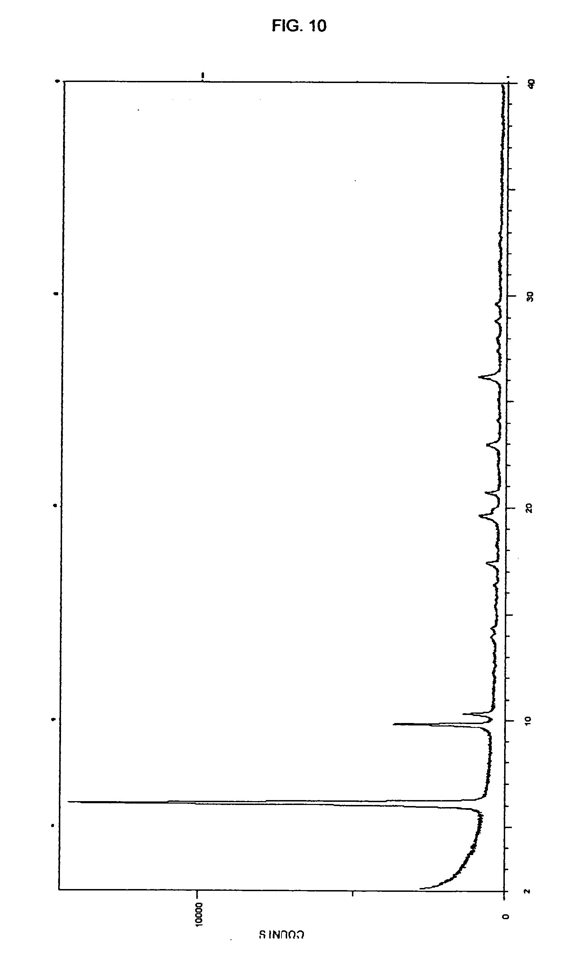

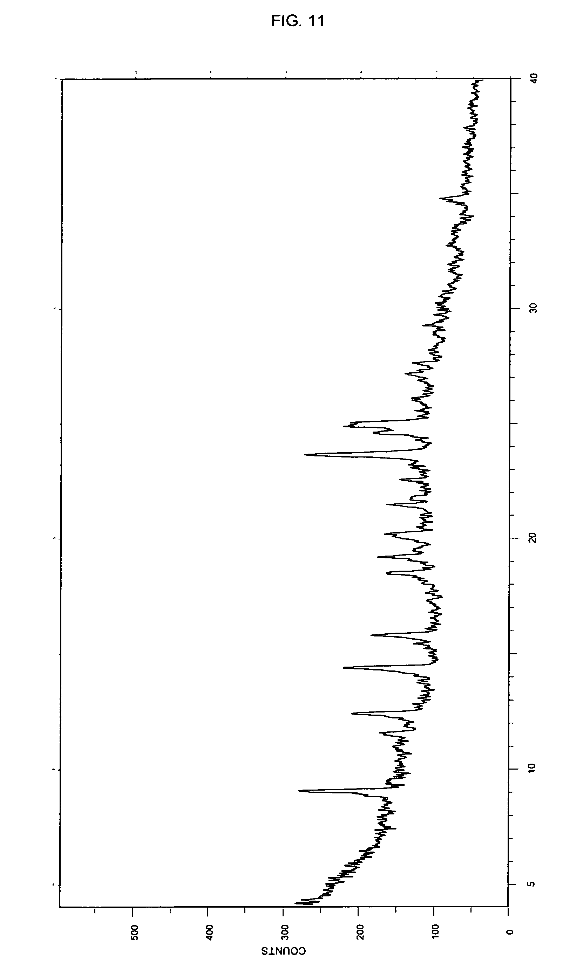

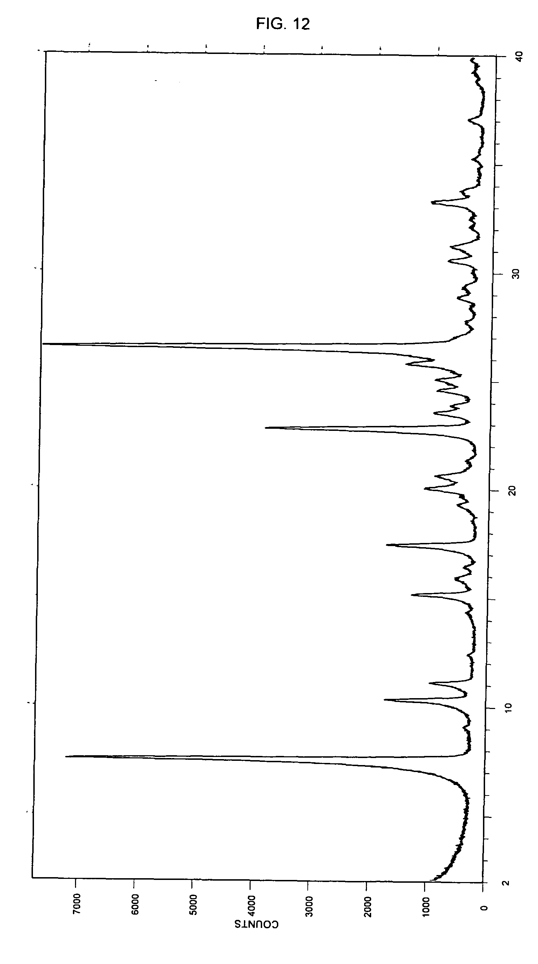

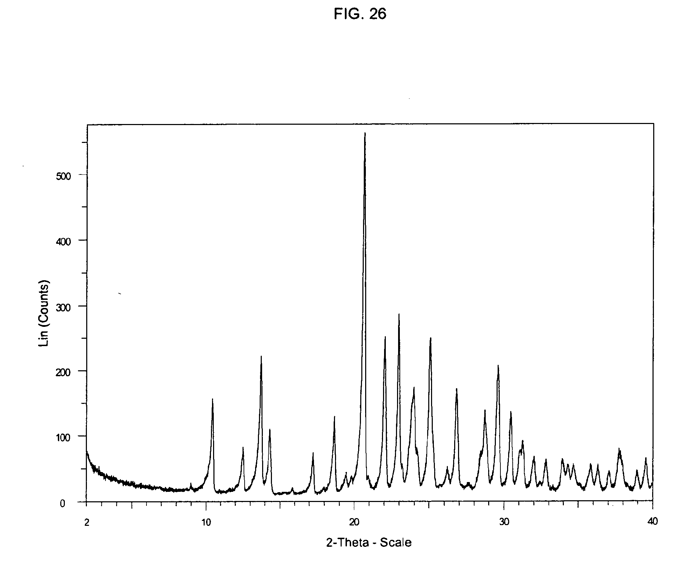

Powder diffraction data were collected on a Siemens D5000 Powder X-ray diffractometer equipped with a Goebel mirror. The powder diffractograms recorded are shown in Figures 7 to 12 and 26. A conventional sample holder was used for all probes with a minimum amount (-100 mg) of substance whereas for Form D a silicone holder was used. Therefore, the peak intensities are relatively low in the powder pattern of Form D when compared to other forms.

The most significant peaks and intensities are summarized in Table 4 here below:

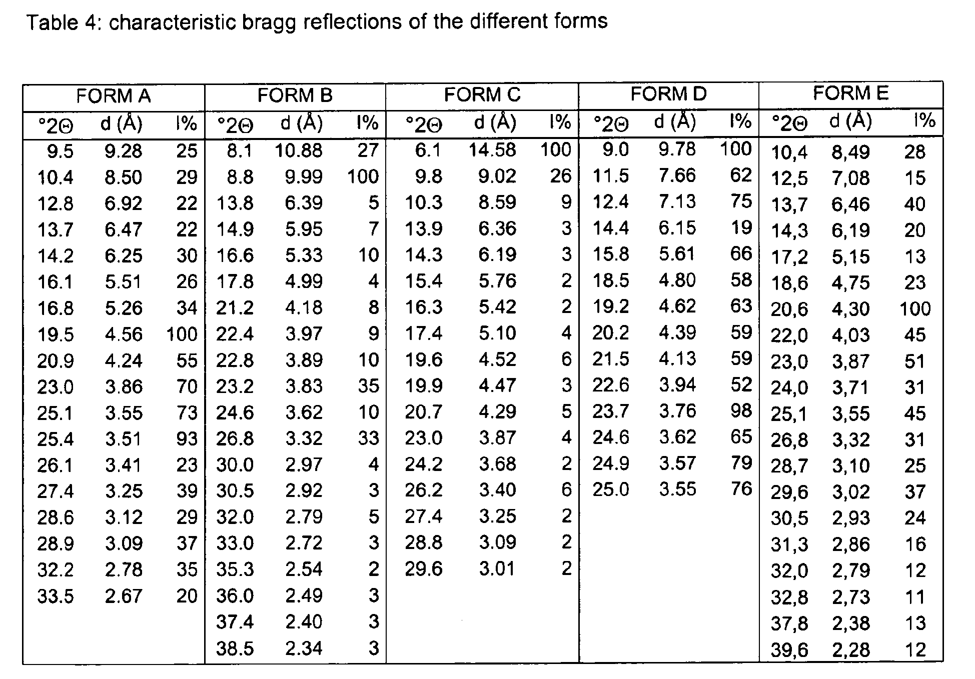

**Table 4b:**

| HY-1 | | | HY-2 | | |
|---|---|---|---|---|---|
| °2Θ | d (Å) | I% | °2Θ | d (Å) | I% |
| 9.0 | 9.87 | 19 | 7.5 | 11.71 | 93 |
| 9.7 | 9.08 | 16 | 10.3 | 8.60 | 22 |
| 12.5 | 7.09 | 74 | 11.1 | 7.99 | 12 |
| 13.8 | 6.40 | 18 | 15.1 | 5.85 | 17 |
| 15.9 | 5.58 | 49 | 15.9 | 5.57 | 7 |
| 16.7 | 5.31 | 19 | 16.4 | 5.39 | 5 |
| 18.0 | 4.93 | 22 | 17.4 | 5.09 | 22 |
| 18.8 | 4.71 | 12 | 19.3 | 4.60 | 7 |
| 20.0 | 4.43 | 100 | 20.0 | 4.43 | 14 |
| 20.5 | 4.33 | 51 | 20.6 | 4.30 | 12 |
| 21.5 | 4.13 | 23 | 22.8 | 3.90 | 50 |
| 21.9 | 4.05 | 79 | 23.6 | 3.77 | 12 |
| 23.3 | 3.81 | 25 | 23.9 | 3.72 | 9 |
| 24.2 | 3.68 | 39 | 24.6 | 3.62 | 11 |
| 24.6 | 3.61 | 23 | 25.1 | 3.55 | 12 |
| 26.2 | 3.40 | 36 | 25.8 | 3.45 | 19 |
| 27.9 | 3.20 | 56 | 26.5 | 3.35 | 100 |
| 28.4 | 3.14 | 56 | 28.9 | 3.09 | 7 |
| 30.3 | 2.95 | 19 | 29.3 | 3.04 | 6 |
| 32.1 | 2.79 | 31 | 30.6 | 2.92 | 10 |
| 33.3 | 2.69 | 29 | 31.3 | 2.86 | 9 |
| 34.9 | 2.57 | 21 | 33.3 | 2.69 | 13 |
| 36.4 | 2.46 | 21 | 33.8 | 2.65 | 7 |
| 37.8 | 2.38 | 22 | 37.1 | 2.42 | 6 |

### Example 13: FT-Raman Spectrometry

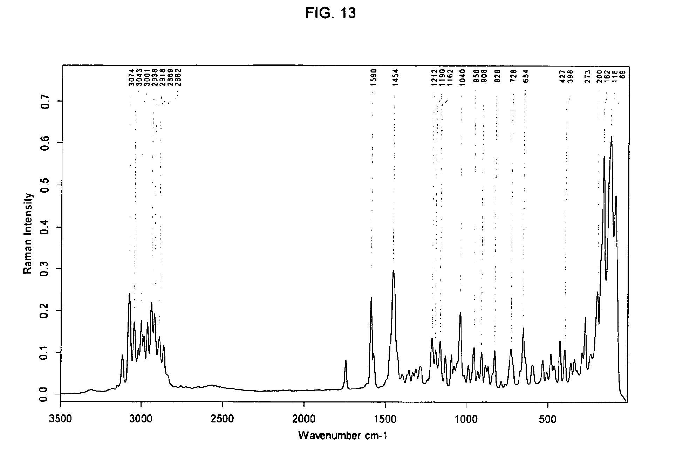

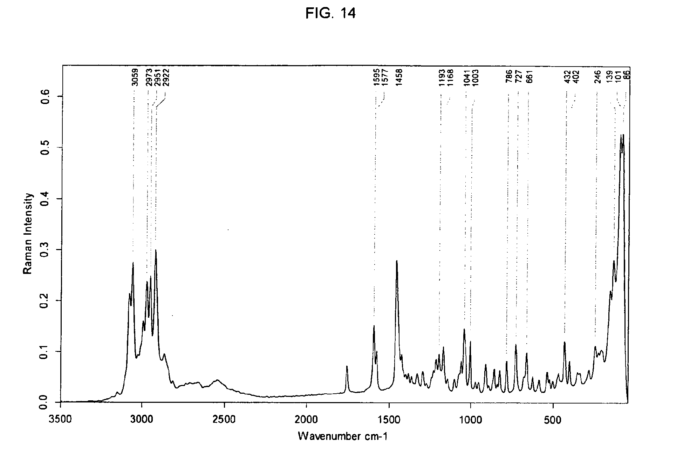

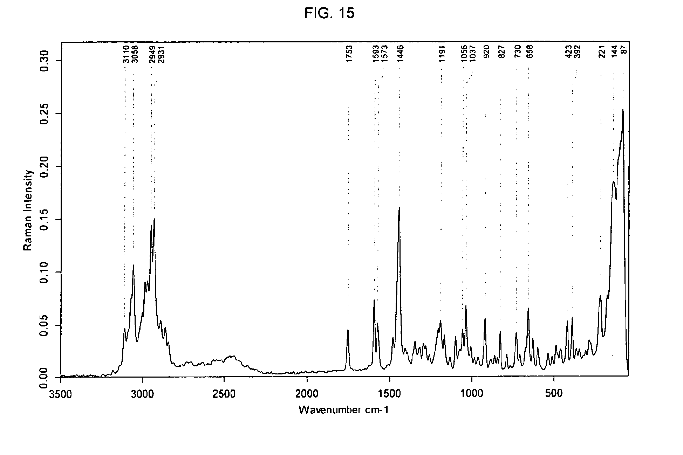

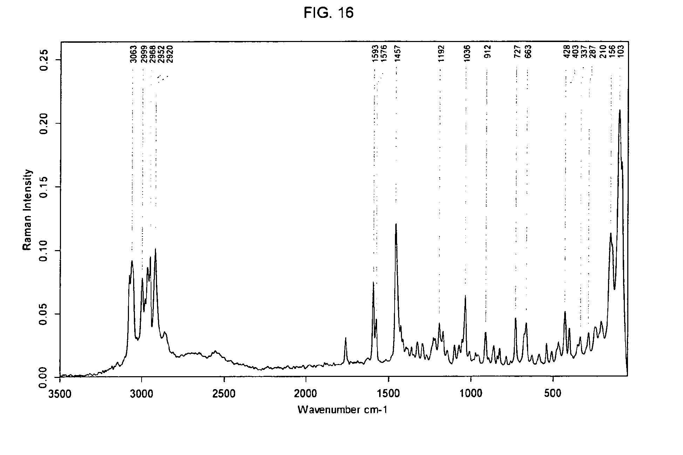

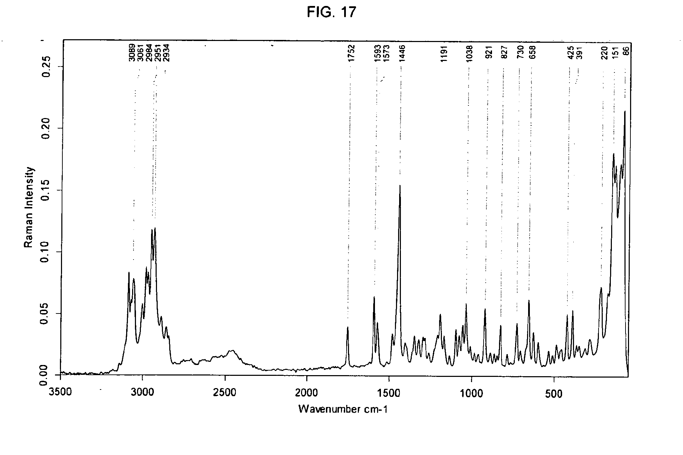

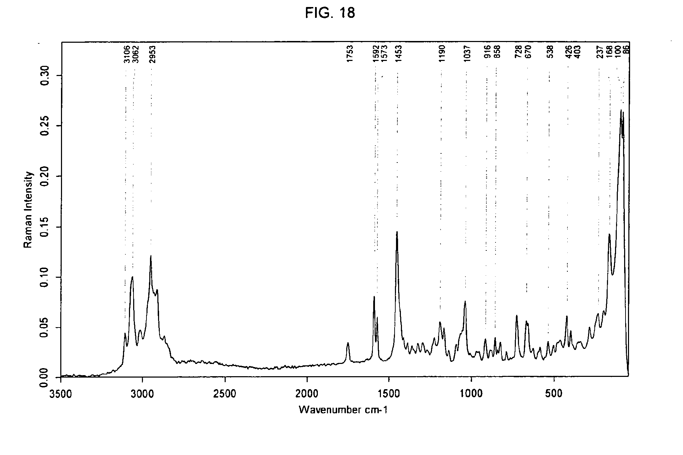

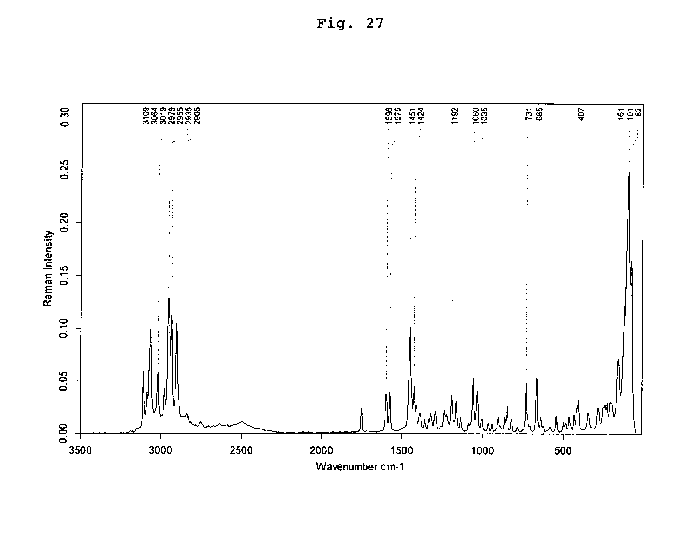

Raman spectral data were collected on a Bruker RFS 100 Raman-spectrometer. The samples were obtained by grinding the crystals/powders of the respective modifications. The spectra recorded are shown in Figures 13 to 18 and 27. The most significant difference that makes these polymorphs distinguishable is the wave number region of 2800-3200 cm⁻¹ (ν_{C-H} stretching vibrations).

Some significant peaks and intensities are summarized in Table 5 here below:

**Table 5: characteristic raman-bands**

| | | 1 | 2 | 3 | 4 | 5 | 6 | 7 | 8 | 9 | 10 |
|---|---|---|---|---|---|---|---|---|---|---|---|
| A | [cm⁻¹] | 3110 | 3058 | 2949 | 2931 | 1753 | 1593 | 1446 | 1037 | 920 | 658 |
| B | [cm⁻¹] | 3063 | 2999 | 2968 | 2952 | 2920 | 1593 | 1457 | 1036 | 727 | 663 |
| C | [cm⁻¹] | 3089 | 3061 | 2984 | 2951 | 2934 | 1752 | 1593 | 1446 | 1038 | 658 |
| D | [cm⁻¹] | 3106 | 3062 | 2953 | 1753 | 1592 | 1453 | 1037 | 858 | 728 | 670 |
| E | [cm⁻¹] | 3109 | 3064 | 3019 | 2955 | 2905 | 1596 | 1451 | 1060 | 731 | 665 |
| HY-1 | [cm⁻¹] | 3074 | 3043 | 3001 | 2938 | 2918 | 1590 | 1454 | 1040 | 828 | 654 |
| HY-2 | [cm⁻¹] | 3059 | 2973 | 2951 | 2922 | 1595 | 1458 | 1041 | 1003 | 727 | 661 |

### Example 14: Single Crystal X-ray Diffraction

### a) crystal structure determination of modifications HY-1, A and B

Single crystal X-ray data of hydrate HY-1 and anhydrous forms A and B were collected on a STOE image plate diffractometer, using Mo-Kα radiation. The structure solution and refinement were carried out using SHELXL programs built in with the SHELXTL (Version 6.12) package. All non-hydrogen atoms were refined anisotropically. The positions of all the H-atoms bound to phenyl groups, methyl groups and methylene groups in Forms HY-1, A and B were generated by a riding model on idealized geometries with Uiso(H) = 1.2 Ueq(C), while the H-atoms of the tertiary ammonium ions in HY-1, A and B were located from different Fourier maps and were refined isotropically. The hydrogen atoms of the water molecule in HY-1 were also taken from Fourier maps and refined isotropically. The details of the X-ray data collection, structure solution, and refinement are given in the supporting information below.

### b) crystal structure of Form HY-1 (hydrate)

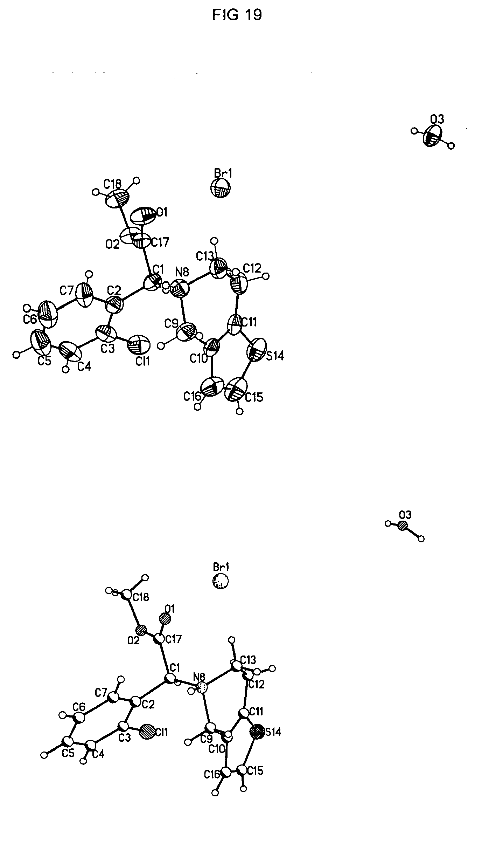

The long thin plate like hydrate crystals (HY-1) crystallised from H₂O were selected for data collection. It crystallises in orthorhombic, chiral space group, *P*2₁2₁2₁. The asymmetric unit consists of one molecule of the clopidogrel hydrogenbromide salt [CG+:BR-] and one molecule water (Figure 19).

The position parameters are summarized in Table 6 here below:

**Table 6:**

| | x | y | z | U(eq) |
|---|---|---|---|---|
| Br(1) | 8958(1) | 4173(1) | 1976(1) | 57(1) |
| Cl(1) | 5055(2) | 7079(2) | -360(1) | 57(1) |
| O(1) | 5548(5) | 6719(5) | 2343(3) | 56(1) |
| O(2) | 7075(5) | 7313(5) | 1485(2) | 50(1) |
| O(3) | 11720(6) | 3824(6) | 2399(3) | 57(1) |
| C(1) | 4827(7) | 6456(6) | 1164(3) | 36(1) |
| C (2) | 4127(7) | 7729(6) | 911(3) | 41(2) |
| C (3) | 4219(7) | 8102(7) | 227(4) | 45(2) |
| C (4) | 3660(7) | 9300(8) | 16(4) | 57(2) |
| C (5) | 3007(9) | 10127(8) | 472(6) | 71(3) |
| C (6) | 2874(9) | 9756(8) | 1144(5) | 68(2) |
| C (7) | 3439(8) | 8596(7) | 1363(5) | 53(2) |
| N (8) | 3690(6) | 5489(5) | 1407(3) | 40(1) |
| C (9) | 2482(7) | 5254(7) | 894(4) | 44(2) |
| C (10) | 3091(7) | 4434(6) | 319(3) | 40(2) |
| C (11) | 5150(9) | 3512(7) | 1033(4) | 55(2) |
| C (12) | 5150(9) | 3512(7) | 1033(4) | 55(2) |
| C (13) | 4355(8) | 4203(7) | 1618(4) | 52(2) |
| S (14) | 4707(2) | 2863(2) | -355(1) | 60(1) |
| C (15) | 3303(10) | 3606(8) | -754(4) | 62(2) |
| C(16) | 2514(8) | 4420(8) | -358(4) | 58(2) |
| C (17) | 5850(7) | 6837(6) | 1746(4) | 40(2) |
| C (18) | 8169(8) | 7827(8) | 1966(4) | 59(2) |

### c) crystal structure of Form A (anhydrous)

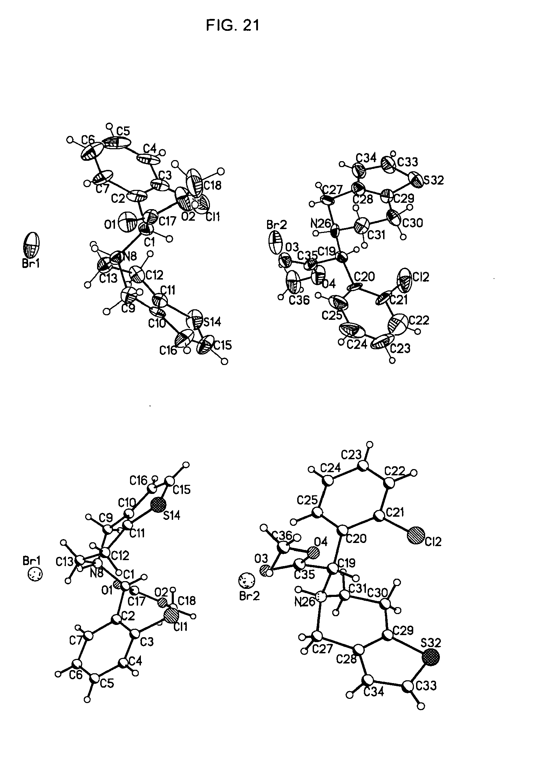

The thin rod like Form A crystals from an ethylacetate/methanol (9:1) batch were selected for data collection. It crystallises in monoclinic, non-centrosymmetric space group, *P*2₁ with two molecules in asymmetric unit (Figure 21). All the three structures are ionic bromides [CG+:BR-], where the acidic hydrogen of the hydrobromic acid has been transferred to a tertiary nitrogen atom of clopidogrel (CG). The atomic numbering, given in the ortep diagram is similar for all the three structures (Figure 21).

The position parameters are summarized in Table 7 here below:

**Table 7:**

| | x | y | z | U(eq) |
|---|---|---|---|---|
| Br(1) | 894(2) | 6851(1) | -816(1) | 80(1) |
| Br (2) | 3357(2) | 6456(2) | 4215(1) | 86(1) |
| Cl (1) | 4131(5) | 6841(7) | 2457(2) | 96(2) |
| Cl (2) | 3074(8) | 6445(8) | 7432(3) | 142(3) |
| O (1) | 2235(12) | 9689(10) | 312(5) | 66(3) |
| O (2) | 3560(15) | 10062(12) | 1284(5) | 88(4) |
| O (3) | 3094(11) | 3608(10) | 5322(5) | 61(3) |
| O (4) | 2839(14) | 3134(12) | 6309(5) | 84(4) |
| C (1) | 2415(15) | 7786(15) | 1109(6 | 48(4) |
| C (2) | 3785(15) | 6825(18) | 1169(6) | 58(4) |
| C (3) | 4571(15) | 6310(17) | 1752(7) | 56(4) |
| C (4) | 5684(19) | 5340(20) | 1765(12) | 86(6) |
| C (5) | 6090(20) | 4901(18) | 1223(16) | 102(9) |
| C (6) | 5350(20) | 5420(20) | 633(11) | 93(6) |
| C (7) | 4233(18) | 6410(20) | 606(8) | 77(5) |
| N (8) | 1116(13) | 7140(12) | 688(6) | 45(3) |
| C (9) | -310(17) | 7894(15) | 728(6) | 52(4) |
| C (10) | -732(14) | 7571(15) | 1361(6) | 43(4) |
| C (11) | -161(14) | 6375(16) | 1700(5) | 47(3) |
| C (12) | 818(17) | 5261(14) | 1512(6) | 53(4) |
| C (13) | 855(18) | 5509(15) | 820(7) | 55(4) |
| S (14) | -666(6) | 6367(5) | 2431(2) | 80(1) |
| C (15) | -1660(20) | 7889(16) | 2250(9) | 79(6) |
| C (16) | -1630(20) | 8464(18) | 1658(8) | 75(5) |
| C (17) | 2705(17) | 9284(15) | 843(7) | 50(4) |
| C (18) | 3960(30) | 11520(30) | 1099(9) | 139(10) |
| C(19) | 3610(15) | 5486(15) | 6123(6) | 50(4) |
| C (20) | 2248(12) | 6415(14) | 6124(7) | 74(5) |
| C (21) | 2013(16) | 6899(17) | 6704(6) | 120(10) |
| C (22) | 830(19) | 7803(16) | 6725(9) | 168(17) |
| C (23) | -117(15) | 8224(14) | 6168(12) | 220(30) |
| C (24) | 117(14) | 7740(18) | 5588(9) | 159(14) |
| C (25) | 1300(15) | 6836(17) | 5567(6) | 116(8) |
| N (26) | 4615(14) | 6186(13) | 5730(6) | 51(3) |
| C (27) | 6060(15) | 5393(17) | 5806(7) | 53(4) |
| C (28) | 6921(17) | 5812(17) | 6478(7) | 56(4) |
| C (29) | 6657(17) | 7048(16) | 6768(6) | 52(4) |
| C (30) | 5505(18) | 8131(15) | 6522(7) | 62(4) |
| C (31) | 4830(20) | 7838(15) | 5819(7) | 58(4) |
| S (32) | 7804(5) | 7105(6) | 7519(2) | 81(2) |
| C (33) | 8560(20) | 5485(19) | 7398(9) | 84(6) |
| C (34) | 8030(20) | 4872(18) | 6821(8) | 72(5) |
| C (35) | 3148(16) | 3973(16) | 5860(7) | 52(4) |
| C (36) | 2410(20) | 1676(19) | 6135(9) | 114(7) |

### d) crystal structure of Form B (anhydrous)

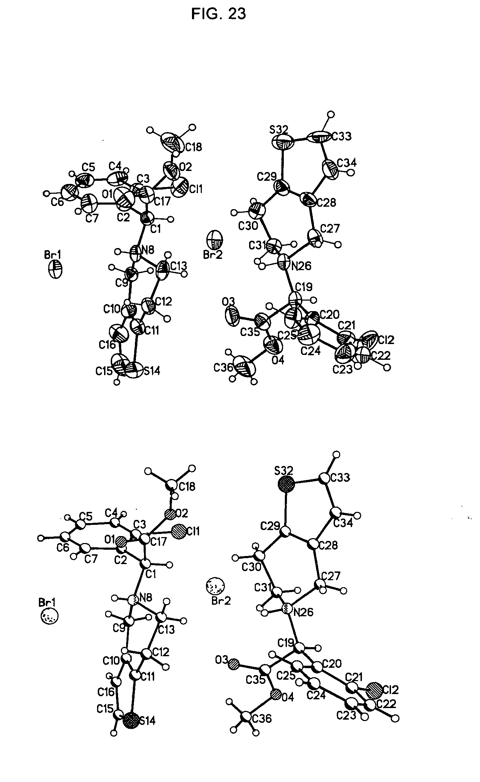

The thin needle like Form B crystals crystallised from a chloroform/ethylacetate (1:3) mixture were selected for data collection. It crystallises in triclinic, non centrosymmetric space group P₁ with again two molecules of clopidogrel hydrogen bromide salt [CG+:BR-] (Figure 23). Unlike the crystal structure of Form A the chiral centres in Form B crystal (molecule 1: C1 and N8; molecule 2: C19 and N26) are (S, R) type for both the molecules (Figure 23).

The position parameters are summarized in Table 8 here below:

**Table 8:**

| | x | y | z | U(eq) |
|---|---|---|---|---|
| Br (1) | 12425(1) | 9485(1) | 5144(1) | 60(1) |
| Br(2) | 7441(1) | 4654(1) | 7756(1) | 61(1) |
| Cl (1) | 4260(4) | 9404(5) | 2011(3) | 116(2) |
| Cl (2) | -812(4) | 4734(5) | 10900(4) | 118(2) |
| O (1) | 9835(11) | 7191(8) | 4411(9) | 93(3) |
| O (2) | 7222(13) | 6723(11) | 3754(12) | 92(3) |
| O (3) | 4790(11) | 6963(8) | 8479(9) | 91(3) |
| O (4) | 2160(13) | 7366(11) | 9184(12) | 93(3) |
| C(1) | 7372(15) | 8646(13) | 3900(11) | 52(3) |
| C (2) | 7777(14) | 9640(14) | 2587(13) | 56(3) |
| C (3) | 6425(13) | 10048(11) | 1685(12) | 65(3) |
| C (4) | 6810(20) | 10975(16) | 508(13) | 84(5) |
| C (5) | 8530(20) | 11464(15) | 256(13) | 83(4) |
| C (6) | 9865(17) | 11058(12) | 1106(10) | 81(4) |
| C (7) | 9506(16) | 10130(10) | 2253(10) | 72(3) |
| N (8) | 8037(12) | 9058(7) | 4855(7) | 50(2) |
| C (9) | 7364(15) | 10344(12) | 4608(12) | 51(3) |
| C(10) | 7622(16) | 10608(14) | 5698(14) | 64(4) |
| C(11) | 7974(14) | 9737(12) | 6873(11) | 66(3) |
| C (12) | 8315(14) | 8409(11) | 7122(10) | 69(3) |
| C (13) | 7410(17) | 8127(15) | 6127(12) | 66(4) |
| S (14) | 8065(5) | 10340(5) | 7935(4) | 105(1) |
| C (15) | 7570(30) | 11810(20) | 6780(30) | 132(9) |
| C (16) | 7410(20) | 11840(20) | 5650(20) | 104(6) |
| C (17) | 8289(14) | 7429(11) | 4063(10) | 65(3) |
| C (18) | 8020(20) | 5553(18) | 3810(20) | 134(7) |
| C(19) | 2367(14) | 5453(13) | 9031(12) | 54(3) |
| C (20) | 2708(15) | 4487(13) | 10321(11) | 51(3) |
| C (21) | 1370(14) | 4099(13) | 11224(12) | 71(3) |
| C (22) | 1790(20) | 3147(15) | 12384(13) | 86(5) |
| C (23) | 3470(20) | 2656(15) | 12650(13) | 83(4) |
| C (24) | 4840(18) | 3058(12) | 11796(11) | 84(4) |
| C (25) | 4460(14) | 3988(11) | 10648(10) | 68(3) |
| N (26) | 3060(10) | 5093(7) | 8039(7) | 45(2) |
| C (27) | 2396(17) | 3770(13) | 8259(14) | 61(4) |
| C (28) | 2624(14) | 3509(15) | 7167(15) | 63(4) |
| C (29) | 3042(15) | 4418(12) | 6042(12) | 68(3) |
| C(30) | 3355(14) | 5761(11) | 5784(10) | 65(3) |
| C (31) | 2428(16) | 6019(13) | 6790(11) | 52(3) |
| S (32) | 3090(5) | 3827(5) | 4953(4) | 102(1) |
| C (33) | 2560(20) | 2360(30) | 5990(20) | 118(9) |
| C (34) | 2380(20) | 2319(16) | 7126(17) | 85(5) |
| C (35) | 3281(15) | 6702(10) | 8849(10) | 60(3) |
| C (36) | 2930(20) | 8569(17) | 9080(20) | 128(7) |

**Table 9: Crystallographic data for polymorphs of clopidogrel hydrobromide (hydrate, HY-1 and anhydrous forms A, B)**

| | HY-1 | A | B |
|---|---|---|---|
| empirical formula | C₁₆H₁₉NO₃SClBr | C₁₆H₁₇NO₂SClBr | C₁₆H₁₇NO₂SClBr |
| molecular weight | 420.74 | 402.73 | 402.73 |
| crystal system | orthorhombic | monoclinic | triclinic |
| space group | *P*2₁2₁2₁ | *P*2₁ | *P*1 |
| T/(K)^{a} | 293(2) | 293(2) | 293(2) |
| a/Å | 9.0755(18) | 9.341(5) | 7.333(5) |
| b/A | 10.231(2) | 9.168(5) | 11.799(5) |
| c/A | 19.752(4) | 21.626(5) | 12.004(5) |
| α/° | 90 | 90 | 65.226(5) |
| β/° | 90 | 102.290(5) | 89.428(5) |
| γ/° | 90 | 90 | 89.433(5) |
| Z | 4 | 4 | 2 |
| V/Å³ | 1834.1(6) | 1809.6(14) | 943.0(9) |
| Dcalc/g cm⁻³ | 1.524 | 1.478 | 1.418 |
| Reflections collected | 8847 | 8777 | 4551 |
| Unique reflections | 2386 | 4696 | 4022 |
| Observed reflections | 2218 | 3180 | 3511 |
| R1[I>2σ(I)] | 0.0488 | 0.0783 | 0.0462 |
| wR2(all) | 0.1217 | 0.1888 | 0.1230 |
| Goodness-of-fit | 1.019 | 1.067 | 1.001 |
| Ck*^{b} | 66.9 | 64.1 | 61.4 |

| | | | |
|---|---|---|---|
| ^{a} temperature of data collection | | | |
| ^{b} Ck* is the packing fraction calculated with the program PLATON | | | |
| ^{c} the onset melting point calculated from the DSC experiment | | | |

### Methods and Devices:

**Differential scanning calorimetry** (DSC) was performed with a DSC 7 (Perkin-Elmer, Norwalk, Ct., USA) using the Pyris 2.0 software. Approximately 1 to 3 ± 0.0005 mg sample (using a UM3 ultramicrobalance, Mettler, Greifensee, CH) were weighed into Al-Pans (25 µl). Dry nitrogen was used as the purge gas (purge: 20 ml min⁻¹). The instrument was calibrated for temperature with pure benzophenone (m.p. 48.0°C) and caffeine (m.p. 236.2°C) and the energy calibration was performed with pure indium (purity 99.999%, m.p. 156.6°C, heat of fusion 28.45 J. g⁻¹).

Thermogravimetry: Thermogravimetrie-System TGA-7, Pyris-Software for Windows NT, (Perkin-Elmer, Norwalk, Ct., USA), sample amount about 2 to 4 mg. Platinum sample container (50 µl), nitrogen as flushing gas (sample purge: 20 mL min⁻¹, balance purge: 40 mL min⁻¹). Heating rate 5 K min⁻¹.

**Raman spectra** were recorded with a Bruker RFS 100 Raman-spectrometer (Bruker Analytische Messtechnik GmbH, Karlsruhe, D), equipped with a Nd:YAG Laser (1064 nm) as the excitation source and a liquid-nitrogen-cooled, high sensitivity Ge-detector. The spectra were recorded in aluminum sample holders with a laser power of 100 mW (64 scans per spectum) and a resolution of 4 cm⁻¹.

**The powder X-ray diffraction patterns** (PXRD) were obtained with a Siemens D-5000 diffractometer (Siemens AG, Karlsruhe, Germany) equipped with theta/theta goniometer, a CuK_{α} radiation source, a Goebel mirror (Bruker AXS, Karlsruhe, Germany), a 0.15° soller slit collimator and a scintillation counter. The patterns were recorded at a tube voltage of 40 kV and a tube current of 35 mA, applying a scan rate of 0.005° 2Θs⁻¹ in the angular range of 2 to 40° (2Θ). The accuracy of conventional recorded powder X-ray diffraction patterns is generally ± 0.2°.

## Claims

1. Polymorph of clopidogrel hydrobromide selected from the group consisting of Form A, Form B, Form C, Form D, Form E and a hydrated form, wherein
- Form A has a PXRD pattern with peaks at about 19.5, 25.4, 25.1, 23.0 and 20.9;
- Form B has a PXRD pattern with peaks at about 8.1, 8.8, 23.2, 724.6, 26.8, 22.8 and 16.6;
- Form C has a PXRD pattern with peaks at about 6.1, 9.8 and 10.3;
- Form D has a PXRD pattern with peaks at about 9.0, 23.7, 24.9 and 14.4; and
- Form E has a PXRD pattern with peaks at about 20.6, 23.0, 22.0 and 25.1.

2. Polymorph according to claim 1, **characterized in that** the hydrated form of clopidogrel hydrobromide is a monohydrate.

3. Polymorph according to claim 1 or 2, **characterized in that** it is a hydrated form of crystalline clopidogrel hydrobromide selected from the group consisting of Form HY-1 and Form HY-2, wherein
- Form HY-1 has a PXRD pattern with peaks at about 12.5, 20.0, 21.9, 27.9 and 28.4;
and
- Form HY-2 has a PXRD pattern with peaks at about 17.4, 22.8, 7.5, 10.3 and 26.5 degrees 2Θ.

4. Polymorph according to claim 3, which is of Form HY-1.

5. Polymorph according to any of the preceding claims as medicament.

6. Pharmaceutical composition comprising a polymorph according to any of claims 1 to 4 and a pharmaceutically acceptable excipient.

7. Use of a polymorph according to any of claims 1 to 4 for the manufacture of a medicament for inhibiting platelet aggregation.

8. Process for the preparation of a polymorph according to any of claims 1 to 4 comprising the steps of
(i) providing a solution of clopidogrel hydrobromide in a solvent; and
(ii) precipitating clopidogrel hydrobromide therefrom.

9. Process according to claim 8, **characterized in that** the solvent is selected from the group consisting of water, aliphatic alcohols, aliphatic nitrils, aliphatic esters, aliphatic or aromatic hydrocarbons, aliphatic halogenated hydrocarbons, aliphatic ethers and mixtures thereof.

10. Process according to claim 9, **characterized in that** the solvent is selected from the group consisting of water, methanol, ethanol, n-propanol, n-butanol, acetonitrile, ethylacetate, toluene, hexane, dichloromethane, chloroform, tetrachloromethane, tetrahydrofurane, diethylether, and mixtures thereof.

11. Process according to claim 9, **characterized in that** the solvent is selected from the group consisting of water, n-propanol/water, n-butanol/water, n-butanol, acetonitrile, toluene, tetrahydrofurane, ethylacetate, n-butanol/hexane, ethylacetate/methanol, chloroform/ ethylacetate, toluene/methanol, tetrachloromethane/dichloromethane, n-butanol/diethylether, chloroform, and ethanol.

12. Process according to any of claims 8 to 11, **characterized in that** step (ii) is initiated by evaporation and/or cooling of the solvent.

13. Process according to any of claims 8 to 11, **characterized in that** step (i) is performed by means of a solvent capable of dissolving clopidogrel hydrobromide and step (ii) is initiated by adding an antisolvent.

14. Process according to claim 13, **characterized in that** the solvent is selected from the group consisting of methanol, ethanol, n-propanol, i-propanol, n-butanol, n-pentanol, acetic acid, dichloromethane, chloroform, acetone, acetonitrile, nitromethane, dioxane, tetrahydrofurane, ethylacetate, toluene, benzene, xylene, dimethylformamide and dimethylsulfoxide and that the antisolvent is selected from the group consisting of tetrachloromethane, petrolether, diethyl ether, n-hexane, cyclohexane and n-decane.
